(19) Europäisches Patentamt
European Patent Office
Office européen des brevets

(11) EP 1 049 703 B1

(12) EUROPEAN PATENT SPECIFICATION

(45) Date of publication and mention of the grant of the patent:
**12.02.2003 Bulletin 2003/07**

(21) Application number: **98958672.2**

(22) Date of filing: **20.11.1998**

(51) Int Cl.[7]: **C07F 9/6512**, C07K 5/02, C07K 5/06

(86) International application number:
**PCT/US98/24848**

(87) International publication number:
**WO 99/036426 (22.07.1999 Gazette 1999/29)**

(54)
# N-[2-(5-BENZYLOXYCARBONYL-AMINO-6-OXO-2-(4-FLUOROPHENYL)-1,6-DIHYDRO-1-PYRIMIDINYL)ACETOXYL]-L-ASPARTIC ACID ALDEHYDE AS AN IN VIVO INHIBITOR OF INTERLEUKIN-1BETA CONVERTING ENZYME

N-[2-(5-BENZYLOXYCARBONYL-AMINO-6-OXO-2-(4-FLUROPHENYL)1,6-DIHYDRO-1-PYRIMIDINYL)ACETOXYL)-L-ASPARAGINSÄURE ALDEHYDE ALS IN VIVO INHIBITOR DES INTERLEUKIN-1BETA KONVERTIERENDEN ENZYMS

ALDEHYDE DE L'ACIDE N-2-(5-BENZYLOXYCARBONYL-AMINO-6-OXO-2-(4-FLUOROPHENYL)-1,6-DIHYDRO-1-PYRIMIDINYL)ACETOXYL]-L-ASPARTIQUE, INHIBITEUR $i(in vivo) DE L'ENZYME DE CONVERSION DE L'INTERLEUKINE-1$g(b)

(84) Designated Contracting States:
**AT BE CH CY DE DK ES FI FR GB GR IE IT LI LU MC NL PT SE**
Designated Extension States:
**AL LT LV MK RO SI**

(30) Priority: **20.01.1998 US 71918 P**

(43) Date of publication of application:
**08.11.2000 Bulletin 2000/45**

(73) Proprietors:
- **WARNER-LAMBERT COMPANY**
  **Morris Plains, New Jersey 07950 (US)**
- **Abbott GmbH & Co. KG**
  **65205 Wiesbaden (DE)**

(72) Inventors:
- **SCHIELKE, Gerald, Paul**
  **Ann Arbor, MI 48103 (US)**
- **SHAHRIPOUR, Aurash**
  **Ann Arbor, MI 48105 (US)**

(74) Representative: **Riedl, Peter, Dr. et al**
**Patentanwälte**
**Reitstötter, Kinzebach & Partner**
**Postfach 86 06 49**
**81633 München (DE)**

(56) References cited:
**WO-A-93/21210** **WO-A-95/26958**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 049 703 B1

## Description

BACKGROUND OF THE INVENTION

### Field Of The Invention

This invention relates to the compound N-[2-(5-benzyloxycarbonyl-amino-6-oxo-2-(4-fluorophenyl)-1,6-dihydro-1-pyrimidinyl)acetoxyl]-L-aspartic acid aldehyde as an inhibitor of the interleukin-1β converting enzyme. This invention also relates to a method of treatment of stroke, inflammatory diseases, septic shock, reperfusion injury, Alzheimer's disease, and shigellosis, and to a pharmaceutically acceptable composition that contains N-[2-(5-benzyloxycarbonyl-amino-6-oxo-2-(4-fluorophenyl)-1,6-dihydro-1-pyrimidinyl)acetoxyl]-1-aspartic acid aldehyde.

### Summary Of The Related Art

Interleukin-1β converting enzyme (ICE or Caspase-1) acts on pro-interleukin-1β (pro-IL-1β) to produce interleukin-1β (IL-1β), which is an inflammatory cytokine (Kostura M.J. et al., *Proc. Nat. Acad. Sci.,* 1989;86:5227-5231 and Black R.A. et al., *FEBS Lett.,* 1989;247:386-391). Several diseases are associated with interleukin-1 activity. Examples of diseases in which interleukin-1 is involved include, but are not limited to, inflammatory diseases such as rheumatoid arthritis and inflammatory bowel disease, and neuroinflammatory disorders such as stroke, multiple sclerosis, and Alzheimer's disease (Dinarello C.A., *Eur. Cytokine Netw.*, 1994;5:517). Other diseases include septic shock, reperfusion injury, and shigellosis.

Agents that modulate IL-1β activity have been shown to have beneficial *in vivo* effects. For example, compounds that are interleukin-1 receptor antagonists have been shown to inhibit ischemic and excitotoxic damage in rat brains (e.g., Relton J.K. et al., *Brain Research Bulletin,* 1992;29:243-246). Additionally, ICE inhibitors were shown to reduce inflammation and pyrexia in rats (Elford P.R. et al., *British Journal of Pharmacology*, 1995;115:601-606).

Inhibitors of ICE can also inhibit other cysteine proteases in the ICE family. Recently, the nomenclature of these cysteine proteases in the ICE family (also known as Caspases with ICE being known as Caspase-1) has been further defined. The following proteases are representative members of this class of enzymes using the nomenclature described in Alnemri et al., *Cell,* 1996;87:171: Caspase-2 (also known as Ich-1); Caspase-3 (also known as CPP32, Yama, and apopain); Caspase-4 (also known as TX, Ich-2, and ICE rel-II); Caspase-5 (also known as ICE rel-III); Caspase-6 (also known as Mch2); Caspase-7 (also known as Mch3); Caspase-8 (also known as FLICE and Mch5); Caspase-9 (also known as ICE-LAP6 and Mch6); Caspase-10 (also known as Mch4). It is recognized that members of this enzyme family play key biological roles in both inflammation and apoptosis (programmed cell death) (Thornberry N.A. et al., *Perspectives in Drug Discovery and Design,* 1994;2:389-399).

In addition to its effects on IL-1β production, ICE has been shown to play a role in the production of the inflammatory mediator interferon-γ (Ghayur et al., *Nature,* 1997;386(6625):619-623). ICE processes the inactive proform of interferon-γ inducing factor (IGIF; Interleukin-18) to active IGIF, a protein that induces production of interferon-γ by T-cells and natural killer cells. Interferon-γ has been implicated in the pathogenesis of diseases such as inflammatory disorders and septic shock. Therefore, ICE inhibitors would be expected to have beneficial effects in such disease states by effects on interferon-γ.

The majority of the ICE inhibitors described in the art are peptide-based (e.g., Dolle R. et al; *J. Med. Chem.*, 1994;37:563). However, the use of pyridone or pyrimidone based peptidomimetic inhibitors has recently been reported (Dolle R. et al; WO 9526958, 1995; Dolle R. et al, *J. Med. Chem.,* 1996;39:2438; and Semple G. et al., *Bioorg. Med. Chem. Lett.,* 1997;7:1337). X-ray crystallography and molecular modeling have shown that pyridone based inhibitors are suitable replacements for $P_2$-$P_3$ found in peptide based inhibitors (Golec J. et al, *Bioorg. Med. Chem. Lett.,* 1997; 7:2181-2186).

WO 9526958 discloses the use of pyrimidone based inhibitors of ICE, such as compound **2**:

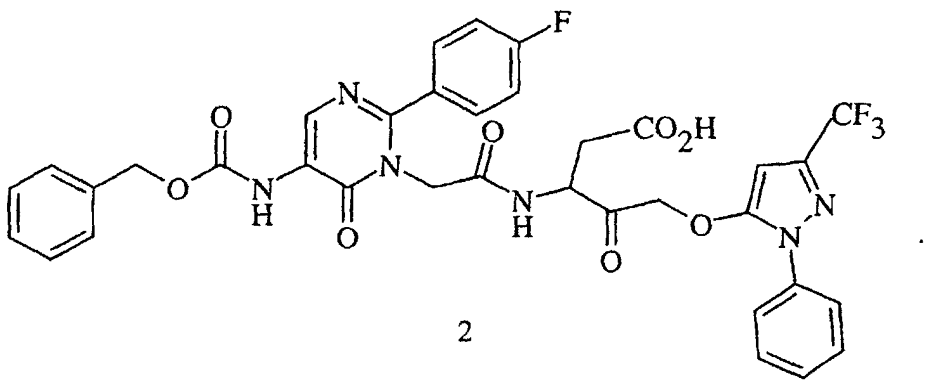

2

As well as possessing some activity in *in vitro* models, the compounds taught in WO 9526958 are reported to have an $IC_{50}$ range of from 0.1 to 10 μm, reflecting the percent inhibition of release of IL-1β. While these values reflect some activity, the search for better ICE inhibitors for the treatment of such diseases as inflammation, Alzheimer's disease, stroke, and septic shock is desired.

SUMMARY OF THE INVENTION

This invention comprises the compound **1** and the stereoisomers and pharmaceutically acceptable salts, esters, amides, and prodrugs thereof:

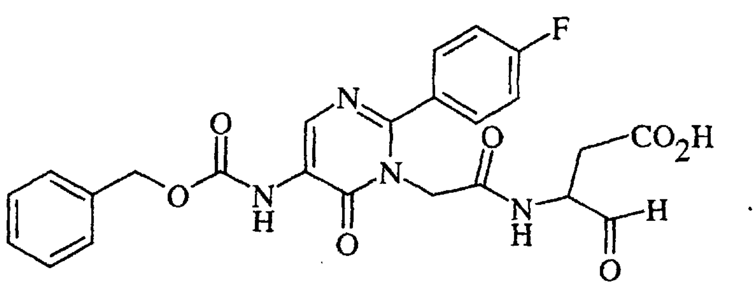

1

Compound **1** shows unexpected superior *in vivo* activity as an inhibitor of ICE compared to prior art ICE inhibitors. In fact, this is the first inhibitor of ICE to show *in vivo* activity in an animal model of stroke after peripheral administration. Prior art compounds such as compound **2** do not show such activity.

DETAILED DESCRIPTION OF THE INVENTION

This invention comprises compound **1** and its use as an inhibitor of the interleukin-1β converting enzyme. This invention also comprises a method of treatment of inflammatory diseases such as arthritis and inflammatory bowel disease, neuroinflammatory disorders such as multiple sclerosis and Alzheimer's disease, and other diseases such as stroke, septic shock, reperfusion injury, and shigellosis, the method comprising administering to a mammal in need of such treatment a therapeutically effective amount of compound **1**. This invention further comprises a pharmaceutically acceptable composition that contains compound **1.** This invention also comprises the use of compound **1** to study the biological effects of inhibiting ICE in *in vitro* and *in vivo* models.

Compound **1,** although a species of the genus disclosed in WO 9526958 ('958 application), has shown unexpectedly good in *vivo* activity as an ICE inhibitor.

A side-by-side comparison of compound **1** and compound **2** (disclosed and claimed in the '958 application) was undertaken. While both compounds show similar activity in enzyme inhibition and in a cell-based test, compound **1** is active in *in vivo* models while compound **2** is not.

Compound **1** significantly blocks production of IL-1β while compound **2** is inactive. Compound **1** also significantly reduces the extent of exitotoxic brain damage in rat pups, a property not seen in compound **2**. Furthermore, mice treated with compound **1** showed reduced arterial lesion size in ischemic brain damage following middle cerebral artery occlusion after reperfusion.

Compound **1** can be administered to a patient either alone or as part of a pharmaceutically acceptable composition. The compositions can be administered to patients such as humans and animals either orally, rectally, parenterally (intravenously, intramuscularly, or subcutaneously), intracisternally, intravaginally, intraperitoneally, intravesically, locally (powders, ointments, or drops), or as a buccal or nasal spray.

Compositions suitable for parenteral injection may comprise physiologically acceptable sterile aqueous or nonaqueous solutions, dispersions, suspensions, or emulsions, and sterile powders for reconstitution into sterile injectable solutions or dispersions. Examples of suitable aqueous and nonaqueous carriers, diluents, solvents or vehicles, include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid; (b) binders, as for example, carboxymethylcellulose, alignates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (e) solution retarders, as for example, paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid compositions of a similar type may also be employed as fillers in soft and hard-filled gelatin capsules using such excipients as lactose or milk sugar, as well as, high molecular weight polyethyleneglycols, and the like.

Solid dosage forms such as tablets, dragees, capsules, pills, and granules can be prepared with coatings and shells, such as enteric coatings and others well-known in the art. They may contain opacifying agents, and can also be of such composition that they release the active compound in a certain part of the intestinal tract in a delayed manner. Examples of embedding compositions which can be used are polymeric substances and waxes. The active compound can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compound, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil and sesame oil, glycerol, tetrahydrofuryl alcohol, polyethyleneglycols, and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compound, may contain suspending agents, as for example, ethoxylated isostearyl alcohols, polyoxyethylene sorbitol and sorbitan esters, microcrystalline cellulose, aluminum metahydroxide, bentonite, agar-agar, and tragacanth, or mixtures of these substances, and the like.

Compositions for rectal administrations are preferably suppositories which can be prepared by mixing compound **1** with suitable non-irritating excipients or carriers such as cocoa butter, polyethyleneglycol, or a suppository wax, which are solid at ordinary temperatures, but liquid at body temperature and therefore, melt in the rectum or vaginal cavity and release the active component.

Dosage forms for topical administration of compound **1** include ointments, powders, sprays, and inhalants. The active component is admixed under sterile conditions with a physiologically acceptable carrier and any preservatives, buffers, or propellants as may be required. Ophthalmic formulations, eye ointments, powders, and solutions are also contemplated as being within the scope of this invention.

Compound **1** can be administered to a patient at dosage levels in the range of about 0.1 to about 1,000 mg

per day. For a normal human adult having a body weight of about 70 kg, a dosage in the range of about 0.01 to about 100 mg per kilogram of body weight per day is preferable. The specific dosage used, however, can vary. For example, the dosage can depend on a numbers of factors including the requirements of the patient, the severity of the condition being treated, and the pharmacological activity of the compound being used. The determination of optimum dosages for a particular patient is well-known to those skilled in the art.

The term "pharmaceutically acceptable salts, esters, amides, and prodrugs" as used herein refers to those carboxylate salts, amino acid addition salts, esters, amides, and prodrugs of the compound of the present invention which are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic and tautomeric forms, where possible, of the compounds of the invention. The term "salts" refers to the relatively nontoxic, inorganic, and organic acid addition salts of compound **1.** These salts can be prepared *in situ* during the final isolation and purification of the compound or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laureate, borate, benzoate, lactate, phosphate, tosylate, citrate, maleate, fumarate, succinate, tartrate, naphthylate mesylate, glucoheptonate, lactobionate and laurylsulphonate salts, and the like. These may include cations based on the alkali and alkaline earth metals, such as sodium, lithium, potassium, calcium, magnesium, and the like, as well as nontoxic ammonium, quaternary ammonium and amine cations including, but not limited to ammonium, tetramethylammonium, tetraethylammonium, methylamine, dimethylamine, trimethylamine, triethylamine, ethylamine, and the like (see, for example, Berge S.M et al., "Pharmaceutical Salts", *J. Pharm. Sci.*, 1977;66:1-19 which is incorporated herein by reference).

Examples of pharmaceutically acceptable, nontoxic esters of compound **1** include $C_1$-$C_6$ alkyl esters wherein the alkyl group is a straight or branched chain. Acceptable esters also include $C_5$-$C_7$ cycloalkyl esters as well as arylalkyl esters such as, but not limited to benzyl. $C_1$-$C_4$ alkyl esters are preferred. Esters of compound **1** may be prepared according to conventional methods.

Examples of pharmaceutically acceptable, nontoxic amides of compound **1** include amides derived from ammonia, primary $C_1$-$C_6$ alkyl amines and secondary $C_1$-$C_6$ dialkyl amines wherein the alkyl groups are straight or branched chain. In the case of secondary amines the amine may also be in the form of a 5- or 6-membered heterocycle containing one nitrogen atom. Amides derived from ammonia, $C_1$-$C_3$ alkyl primary amines, and $C_1$-$C_2$ dialkyl secondary amines are preferred. Amides of compound **1** may be prepared according to conventional methods.

The term "prodrug" refers to compounds that are rapidly transformed *in vivo* to yield the parent compound of compound **1,** for example, by hydrolysis in blood. A thorough discussion is provided in Higuchi T. and Stella V., "Prodrugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987, both of which are incorporated herein by reference.

In addition, compound **1** can exist in unsolvated as well as solvated forms with pharmaceutically acceptable solvents such as water, ethanol, and the like. In general, the solvated forms are considered equivalent to the unsolvated forms for the purposes of the present invention.

Compound **1** is administered to a patient in need of ICE inhibition. In general, patients in need of ICE inhibition are those patients having a disease or condition in which ICE plays a role. Examples of such diseases include, but are not limited to, inflammatory diseases such as rheumatoid arthritis and inflammatory bowel disease and neuroinflammatory disorders such as stroke. Other diseases include reperfusion injury, Alzheimer's disease, and shigellosis.

A "therapeutically effective amount" is an amount of compound **1** that, when administered to a patient having a disease that can be treated with compound **1**, ameliorates a symptom of the disease. A therapeutically effective amount of compound **1** is readily determined by one skilled in the art by administering compound **1** to a patient and observing the results and can be accomplished in a routine manner using art-recognized techniques.

An illustration of the preparation of compound **1** is given in Scheme 1.

Scheme 1

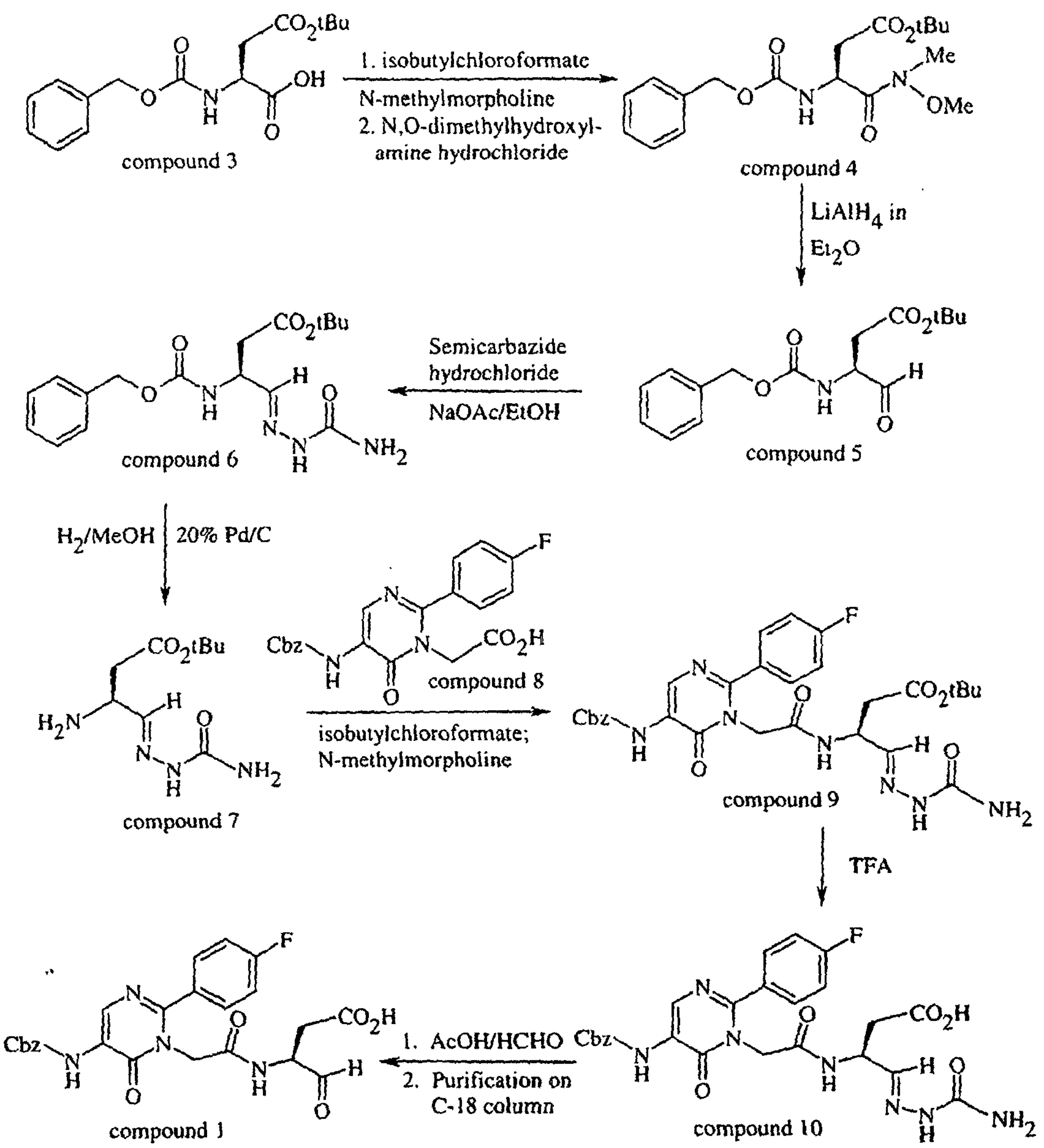


The starting materials and various intermediates may be obtained from commercial sources, prepared from commercially available organic compounds, or routinely prepared using well-known synthetic methods.

The disclosures in this application of all articles and references, including patents, are incorporated herein by reference.

The invention is illustrated further by the following examples which are not to be construed as limiting the invention in scope or spirit to the specific procedures described in them.

EXAMPLE 1

**Preparation of Compound 1**

1.

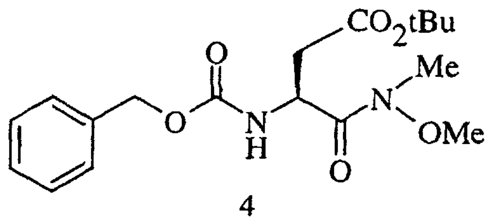

4

To a solution of the acid **3** (50.0 g, 154.6 mmol) in dry dichloromethane (200 mL) and N-methylmorpholine (42.5 mL, 386.5 mmol) at -75°C is added dropwise isobutylchloroformate (24.1 mL, 185.5 mmol) followed by stirring at -75°C for 30 minutes. A solution of *N,O*-dimethylhydroxylamine hydrochloride (18.10 g, 185.5 mmol) in dry dichloromethane (50 mL) and N-methylpiperidine (22.55 mL, 185.5 mmol) is added. The solution is allowed to warm to room temperature and stirred for 1 hour, quenched by addition of 10% $H_2SO_4$ and extracted into ethyl acetate. The organic layer is washed sequentially with 10% $H_2SO_4$, $H_2O$, and brine, dried, and concentrated *in vacuo.* The resulting oil is crystallized upon addition of 20% ether/hexane, and the solid is collected and dried to give the corresponding amide **4** 45.0 g (80%).
$^1$H NMR (400 MHz, $CDCl_3$) $\delta$ 7.30 (5H, s), 5.62 (1H, d), 5.05 (2H, s), 4.95 (1H, m), 3.78 (3H, s), 3.20 (3H, s), 2.65 (1H, dd), 2.55 (1H, dd), 1.39 (9H, s);
MS (APCI) 367 (M+H);
room temperature = 18.2 minutes, gradient elution: 0 to 100 over 20 minutes, 0.10% TFA in $CH_3CN$/water.

2.

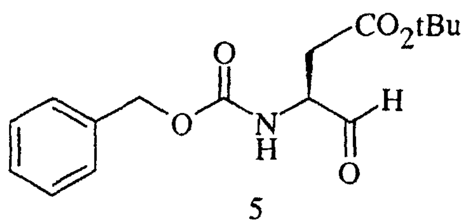

5

To a suspension of lithium aluminum hydride (2.80 g, 74.0 mmol) in dry ether (250 mL) at -75°C is added compound **4** (22.60 g, 61.70 mmol) in $Et_2O$:THF (120 mL, 10:1) dropwise. The resulting solution is stirred at -75°C for 3 hours. The reaction is quenched by dropwise addition of potassium hydrogen sulfate (16.80 g, 123.40 mmol) in water (15 mL) and extracted into ethyl acetate. The organic layer is washed sequentially with $H_2O$ and brine. The solution is dried, and the solvent is removed to provide 5 as an oil 18.0 g (99%).
$^1$H NMR (400 MHz, $CDCl_3$) $\delta$ 9.60 (1H, s), 7.28 (5H, bs), 5.12 (2H, s), 5.03 (1H, s), 2.78-2.58 (2H, m), 1.38 (9H, s);
room temperature =16.9 minutes, gradient elution: 0 to 100 over 20 minutes, 0.10% TFA in $CH_3CN$/water.

3.

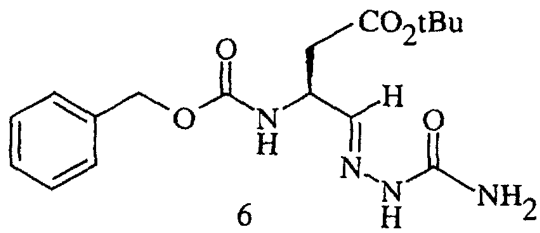

6

To a solution of the aldehyde **5** (21.50 g, 61.40 mmol) in ethanol (200 mL) is added semicarbazide hydrochloride (6.85 g, 61.40 mmol) and sodium acetate (5.10 g, 61.40 mmol) in water (25 mL). The resulting mixture is stirred at room temperature for 5 hours. The ethanol is removed, the crude product is taken into ethyl acetate (300 mL), and the subsequent organic layer is washed with water (150 mL) three times, dried and concentrated to give 21.77 g (97%) of **6** as a foam.
$^1$H NMR (400 MHz, DMSO) δ 9.97 (1H, s), 7.59 (1H, d), 7.23 (5H, m), 7.05 (1H, s), 6.21 (2H, s), 4.98 (2H, s), 4.42 (1H, m), 2.58 (1H, dd), 2.41 (1H, dd), 1.28 (9H, s); MS (APCI) 365.6 (M+H);
room temperature = 15.9 minutes, gradient elution: 0 to 100 over 20 minutes, 0.10% TFA in $CH_3CN$/water.

4.

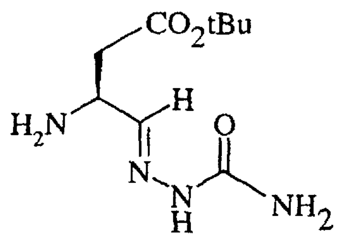

7

To a solution of **6** (2.10 g, 5.80 mmol) in MeOH (20 mL) is added 20% Pd/C (150 mg), and the mixture is stirred for 2 hours under a hydrogen atmosphere at atmospheric pressure. The catalyst is filtered and the solvent removed. The resulting oil solidifies upon the addition of ether. The solid is dried to give 1.15 g (86%) of 7 as an off-white solid. The solid is used immediately in the next step. It is important to solidify this intermediate before submitting to the next step.
$^1$H NMR (400 MHz, DMSO) δ 9.84 (1H, s), 7.02 (1H, d), 6.07 (2H, s), 3.57 (1H, m), 3.38 (2H, m), 2.38 (1H, dd), 2.25 (1H, dd), 1.37 (9H, s);
room temperature = 6.4 minutes, gradient elution: 0 to 100 over 20 minutes, 0.10% TFA in $CH_3CN$/water.

5.

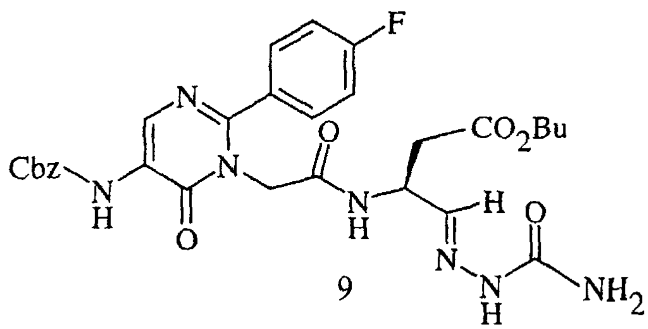

9

To a solution of the acid **7** (6.10 g, 15.30 mmol) and N-methylmorpholine (1.70 mL, 15.30 mmol) in dry dichloromethane (300 mL) at -25°C is added freshly distilled isobutyl chloroformate (2.0 mL, 15.30 mmol), and the resulting solution is stirred for 30 minutes. A second portion of N-methylmorpholine (1.70 mL, 15.30 mmol) is added followed by the amine **8** (3.50 g, 15.30.0 mmol). The solution is maintained at -25°C for 30 minutes and then allowed to warm to room temperature and stir for 1 hour. The solution is filtered free of solids, and the solvent is removed. The resulting residue is dissolved into ethyl acetate (200 mL), washed with water, saturated sodium bicarbonate, dried, and concentrated. The resulting oil is chromatographed (20% THF in dichloromethane) to give 4.60 g (49%) of **9** as a white solid.
$^1$H NMR (400 MHz, DMSO) δ 10.02 (1H, s), 8.87 (1H, s), 8.39 (2H, s), 7.54 (2H, m), 7.34 (8H, m), 7.0 (1H, s), 6.25 (2H, s), 5.13 (2H, s), 4.62 (1H, m), 4.43 (1H, d), 4.36 (1H, d), 2.56 (1H, dd), 2.38 (1H, dd), 1.24 (9H, s);
MS (APCI) 610.3 (M+H);
room temperature = 16.2 minutes, gradient elution: 0 to 100 over 20 minutes, 0.10% TFA in $CH_3CN$/water.

6.

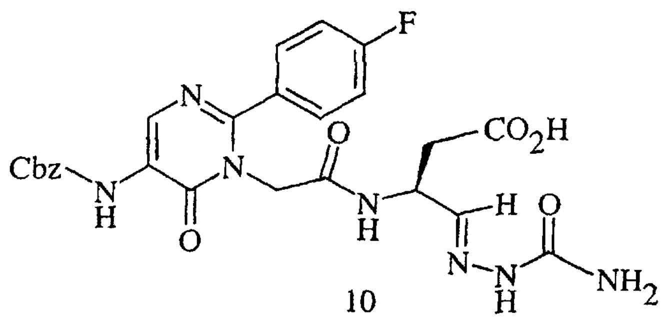

10

To a solution of ester **9** (1.0 g, 1.60 mmol) in dichloromethane (50 mL) at 0°C is added 25% TFA in dichloromethane (20 mL). The resulting solution is allowed to stir at 0°C for 8 hours before it is diluted with toluene (100 mL) and evaporated to provide an oil. The resulting oil is chromatographed (gradient elution: 2.5% MeOH in dichloromethane to 10% MeOH in dichloromethane) to give 0.70 g (79%) of **10** as a white solid.
$^1$H NMR (400 MHz, DMSO) δ 10.03 (1H, s), 8.88 (1H, s), 8.39 (2H, s), 7.50 (2H, m), 7.32 (8H, m), 7.03 (1H, s), 6.20 (2H, bs), 5.12 (2H, s), 4.64 (1H, m), 4.42 (2H, dd), 2.58 (1H, m), 2,42 (1H, m);
MS (APCI) 554.4 (M+H);
room temperature = 12.6 minutes, gradient elution: 0 to 100 over 20 minutes, 0.10% TFA in $CH_3CN$/water.

7.

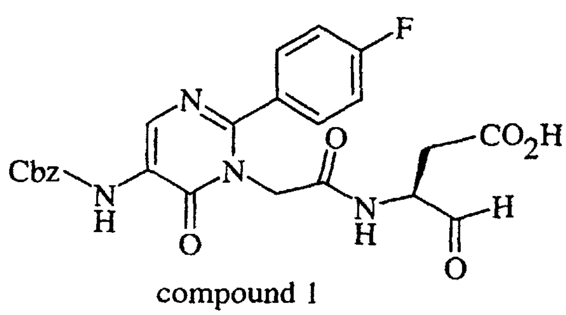

compound 1

To the semicarbazone **10** (6.0 g, 10.80 mmol) is added a solution of 37% aqueous paraformaldehyde and acetic acid (100 mL, 1:1). The reaction mixture is stirred for 2 hours before the solvent is removed, and the resulting oil is diluted with $CH_3CN$ (100 mL) and evaporated. The resulting residue is chromatographed using *mega bond elut* C18 $SiO_2$ (gradient elution: 25% acetonitrile in water to 55% acetonitrile in water) to give 4.20 g (78%) of the final product **1** as a white powder.
$^1$H NMR (400 MHz, $CD_3OD$) δ 8.59 (1H, s), 7.58 (2H, m), 7.35 (7H, m), 5.19 (2H, s), 4.68-4.42 (3H, 4.28 (1H, m), 2.62 (1H, m), 2.37 (1H, m); MS (APCI) 497.4 (M+H);
room temperature = 13.4 minutes, gradient elution: 0 to 100 over 20 minutes, 0.10% TFA in $CH_3CN$/water.

EXAMPLE 2

**In Vitro Assays**

Compounds **1** and **2** are tested for inhibition of ICE as demonstrated by measurement of $K_i$ (µM) and $IC_{50}$ (µM) using the protocol described herein.

1. **Inhibition Studies**

ICE (0.24 nM final concentration) is added to 400 µL of HGDE buffer (100 mM HEPES, 20% glycerol, 5 mM DTT, 0.5 mM EDTA) containing 15 µM substrate (Ac-Tyr-Val-Ala-Asp-AMC; KM = 15 µM) plus vehicle (DMSO) or inhibitor at concentrations bracketing the $K_i$. Substrate hydrolysis is monitored for 300 seconds by observing the fluorescence of released AMC using excitation at 380 nm and emission at 460 nm. Mean rates of substrate hydrolysis are evaluated by linear-regression analysis of the fluorescence vs time traces. To evaluate $K_i$, plots of percent inhibition vs inhibitor concentration are fit by non-linear regression to a reversible, competitive model:

$$\%\text{Inhibition} = \frac{100*[I]}{[I] + K_i\left(1 + \frac{[S]}{KM}\right)}$$

where the competition factor $(1 + [S]/K_M) = 2$.

2. **ICE Colorimetric Dose-Response ($IC_{50}$) Assay**

Diluted inhibitor stocks are prepared by two-fold serial dilution from a primary stock whose concentration is selected (based on screening results or on prior attempts at $IC_{50}$ evaluation) to achieve approximately 95% inhibition in the most concentrated well. Aliquots of each dilution are transferred to a microtitre plate in triplicate.

ICE enzyme is diluted to approximately 24 nM in HGE buffer (100 mM Hepes pH 7.5, 0.5 mM EDTA, 20% glycerol, 0.1% Bovine Serum Albumin (BSA), and activated by adding dithiothreitol (DTT) to a final concentration of 5 mM. The activated enzyme is then aliquoted into wells containing inhibitor or vehicle, and the plate is preincubated for 60 minutes at ambient temperature. Substrate (Ac-Tyr-Val-Ala-Asp-pNA) is added to each well to a final concentration of 50 µM, and plates are placed in the microtitre plate-reader thermostated to 25°°C. Beginning 5 minutes after addition of substrate, absorbance (405 nm) of wells is monitored for 1 hour, and activity is calculated as the mean rate of change in absorbance during this interval.

3. **PBMC Cellular Assay ($IC_{50}$) Determinations**

Further evidence that compound **1** is a potent inhibitor of ICE is provided by its ability to inhibit IL-1β production in human peripheral blood mononuclear cells (PBMCs) as described herein. PBMCs are isolated from heparinized blood by centrifugation over a ficoll cushion, then washed three times with phosphate-buffered saline. PBMCs are suspended in a medium containing RPMI 1640 with glutamine, penicillin, streptomycin, and 2% human AB serum, then plated at $10^6$ cells per well in 96 well flat bottom plates. PBMCs are stimulated overnight with 10 ng/mL of lipopolysaccharide (LPS, *E. Coli* strain 0111:B4; Calbiochem) in the presence or absence of compound **1** or **2**. Medium is harvested, and the level of mature IL-1β was determined using an ELISA kit from R & D Systems. Compound inhibition is assessed by determining the concentration of agent which reduces IL-1β levels by 50%. Cells were cultured for an additional 4 hours in the presence of 3-(4, 5-dimethylthiazol-2-yl)-2, 5-diphenyltetrazolium bromide (MTT) to determine viability. Compound toxicity can, therefore, be assessed by determining the concentration of agent which kills 50% of the cells ($IC_{50}$).

4. **Ich-2 Colorimetric Dose-Response ($IC_{50}$) Assay**

Inhibition of Ich-2 (Caspase 4) enzyme is assayed as described above for ICE, except that enzyme is used at 64 nM, and 60 µM of the Ich-2-specific substrate Ac-Leu-Glu-Val-Asp-pNA is used instead of the ICE substrate Ac-Tyr-Val-Ala-Asp-pNA.

The results of these tests are shown below in Table 1.

Table 1

| Compound | Mol. Wt. | Mode of Inhib. | 1. ICE Ki (μm) | 2. ICE $IC_{50}$ (μm) | 3. PBMC $IC_{50}$ (μm) | 4. Ich-2 $IC_{50}$ (μm) |
|---|---|---|---|---|---|---|
| 1 | 496 | reversible | 0.060 | 0.485 | 1.8 | 9.800 |
| 2 | 737 | irreversible | not determined | 0.004 | 2.2 | 0.005 |

EXAMPLE 3

**In Vivo Models**

1. **Mouse Model**

Female C57BL/6 mice are injected intravenously with 5 mg/kg lipopolysaccaride (LPS) (*E. coli* 0111:B4) at T0; this induces production of the cytokine interleukin-1β. After 3.75 hours from the administration of LPS, the mice are injected intravenously with either vehicle or a test compound. The mice are bled at T4h and plasma analyzed for IL-1β by ELISA. The vehicle is 5% DMSO/20% Trappsol/3% Dextrose/NMDG. Compound **1** shows a 48% inhibition of IL-1β production ($p < 0.03$, student's t-test) when compared to vehicle treated animals. Compound **2** shows an 11% reduction in IL-1β (not significant).

2. **Excitotoxic Brain Damage in Neonatal Rats**

Compound **1** and compound **2** are tested in a model of excitotoxic brain damage in neonatal rats. Seven-day-old rat pups are anesthetized with ether, placed in a head holder and injected with 15 nMoles of MDA into the right caudate nucleus. The pups are treated by intraperitoneal injection with drug (30 mg/kg) or vehicle (0.2% methocel in water) 0.25 hours later and then returned to their home cage. Five days following this excitotoxic challenge, the pups are sacrificed, their brains removed and hemispheres weighed. The loss in weight of the injected hemisphere relative to the contralateral hemisphere is an estimate of the degree of brain necrosis. Table 2 summarizes the results which demonstrate that compound **1** significantly reduces the extent of excitotoxic brain damage, expressed as percent loss of hemisphere weight. Importantly, such protection is not seen at the same dose of compound **2**.

Table 2

| Treatment | % Loss | SD | Number of pups | p-value |
|---|---|---|---|---|
| vehicle | 23.1 | 5.3 | 8 | |
| Compound 1 | 16.1 | 3.9 | 8 | < 0.009 |
| vehicle | 24.6 | 7.2 | 9 | |
| Compound 2 | 20.8 | 3.7 | 8 | <0.26 |

3. **Ischemic Brain Damage Model**

Compound **1** is tested in both permanent (MCAOp) and transient (MCAOt) models of focal cerebral ischemia in the mouse. The middle cerebral artery is occluded by an intraluminal suture in CD-1 mice. In the transient model, the suture is withdrawn after 1 hour and in both studies, mice are sacrificed at 24 hours, and brain lesion size is determined from H and E stained sections. Drug or vehicle is administered IP at -5, 60, and 180 minutes from the time of vessel occlusion at doses of 25 and 50 mg/kg × 3 (MCAOp) or 50 mg/kg × 3 (MCAOt). In the more severe MCAOp, there is a small reduction in lesion size (13%, $p < 0.04$, one-tailed t-test) at the high dose whereas, in the less severe MCAOt, the lesion size is 60% smaller in the drug treated mice ($p < 0.002$).

The invention and the manner and process of making and using it are now described in such full, clear, concise, and exact terms as to enable any person skilled in the art to which it pertains, to make and use the same. It is to be understood that the foregoing describes preferred embodiments of the present invention and that modifications may be made therein without departing from the spirit or scope of the present invention as set forth in the claims. To particularly point out and distinctly claim the subject matter regarded as invention, the following claims conclude this specification.

**Claims**

1. The compound

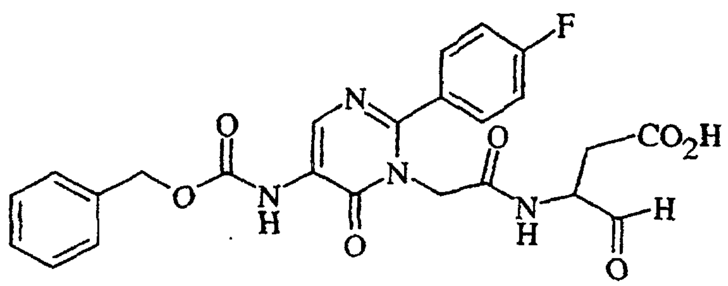

and the stereoisomers and pharmaceutically acceptable salts, esters, amides, and prodrugs thereof.

2. A pharmaceutical composition comprising a therapeutically effective amount of the compound of Claim 1 and a pharmaceutically acceptable carrier.

3. Use of a compound of Claim 1 for the preparation of a pharmaceutical composition for treating interleukin-1β converting enzyme mediated diseases.

4. Use of a compound of Claim 1 for the preparation of a pharmaceutical composition for inhibiting interleukin-1β converting enzyme by contacting the interleukin-1β converting enzyme with said compound.

5. Use of a compound of Claim 1 for the preparation of a pharmaceutical composition for treating or preventing a stroke.

**Patentansprüche**

1. Verbindung

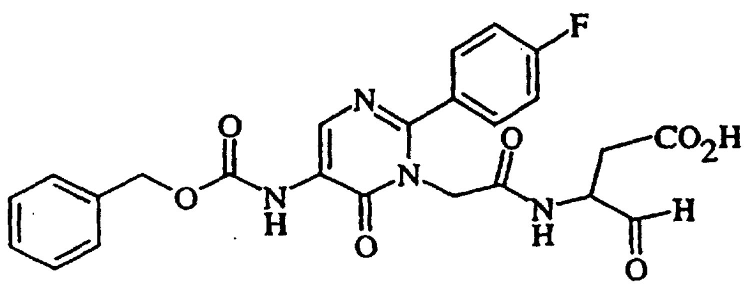

und die Stereoisomere und pharmazeutisch verträglichen Salze, Ester, Amide und Prodrugs davon.

2. Pharmazeutische Zusammensetzung, die eine therapeutisch wirksame Menge der Verbindung nach Anspruch 1 und einen pharmazeutisch verträglichen Träger beinhaltet.

3. Verwendung einer Verbindung nach Anspruch 1 zur Herstellung einer pharmazeutischen Zusammensetzung zur Behandlung von Erkrankungen, die durch ein Interleukin-1β konvertierendes Enzym vermittelt sind.

4. Verwendung einer Verbindung nach Anspruch 1 zur Herstellung einer pharmazeutischen Zusammensetzung zur Inhibierung eines Interleukin-1β konvertierenden Enzyms durch Einwirkung der Verbindung auf das Interleukin-1β konvertierende Enzym.

**5.** Verwendung einer Verbindung nach Anspruch 1 zur Herstellung einer pharmazeutischen Zusammensetzung zur Behandlung oder Prävention eines Gehirnschlags.

## Revendications

**1.** Composé

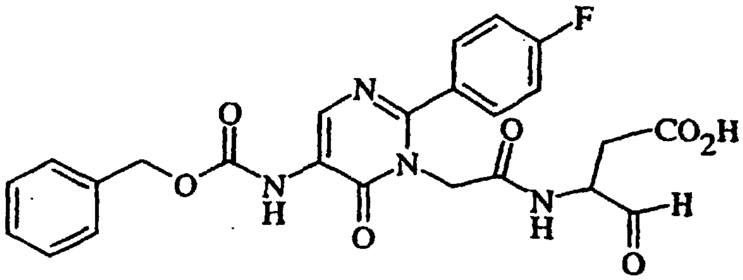

et ses stéréoisomères et ses sels, esters, amides et précurseurs de médicament pharmaceutiquement acceptables.

**2.** Composition pharmaceutique comprenant une quantité thérapeutiquement efficace du composé selon la revendication 1 et un support pharmaceutiquement acceptable.

**3.** Utilisation d'un composé selon la revendication 1 pour la préparation d'une composition pharmaceutique pour le traitement de maladies faisant intervenir l'enzyme de conversion de l'interleukine-1β.

**4.** Utilisation d'un composé selon la revendication 1 pour la préparation d'une composition pharmaceutique pour l'inhibition de l'enzyme de conversion de l'interleukine-1β par mise en contact de l'enzyme de conversion de l'interleukine-1β avec ledit composé.

**5.** Utilisation d'un composé selon la revendication 1 pour la préparation d'une composition pharmaceutique pour le traitement ou la prévention d'un accident vasculaire cérébral.